# FASCICULE DE BREVET EUROPEEN

(11) **EP 4 043 776 B1**
(45) Date de publication et mention de la délivrance du brevet: **11.03.2026**
(21) Numéro de dépôt: 22152141.2
(22) Date de dépôt: 19.01.2022
(51) Int. Cl.: F16M 11/10, F16M 11/20, F16M 13/02, A61M 16/00

(54) **VENTILATEUR MÉDICAL SURMONTÉ D'UN ÉCRAN D AFFICHAGE ORIENTABLE**
MEDIZINISCHES BEATMUNGSGERÄT MIT DREHBAREM BILDSCHIRM
MEDICAL FAN FITTED ON TOP OF AN ADJUSTABLE DISPLAY SCREEN

(30) Priorité: 16.02.2021 FR 2101451; 31.03.2021 FR 2103346
(43) Date de publication de la demande: 17.08.2022
(73) Titulaire: Air Liquide Medical Systems, 92182 Antony Cedex (FR)
(72) Inventeur: LOPEZ, Julien, 92182 Antony Cedex (FR); JOIGNET, Hélène, 92182 Antony Cedex (FR); GIRISH, Rohith, 92182 Antony Cedex (FR)
(74) Mandataire: Air Liquide

(56) Documents cités:
- EP-A1- 3 427 771
- WO-A1-2016/043644
- WO-A1-2019/175221
- DE-B3- 102008 026 989
- US-A1- 2010 148 458
- US-A1- 2011 132 368
- US-B1- 6 669 639

## Description

L'invention concerne un ventilateur médical équipé d'un écran d'affichage d'informations ou autres, agencé de manière orientable sur un bras-porteur formant potence, lequel est fixé à un châssis-support interne du ventilateur.

Un ventilateur médical est un appareil d'assistance respiratoire servant à délivrer une aide respiratoire, c'est-à-dire une ventilation artificielle, à un patient souffrant de troubles ou insuffisances respiratoires, plus ou moins sévères, pouvant résulter de différentes pathologies ou analogues. Certains patients ayant des pathologies sévères peuvent rester ventilés en milieu hospitalier, pendant plusieurs jours, même plusieurs mois.

Pendant son fonctionnement, le ventilateur médical délivre un gaz respiratoire au patient, par exemple de l'air ou de l'air enrichi en oxygène, et opère un suivi, i.e. monitorage, de paramètres ventilatoires (e.g. pression du gaz, pression œsophagienne, volumes de gaz échangés, débit de gaz...). Le gaz respiratoire peut être délivré par une soufflante motorisée, aussi appelée turbine ou compresseur.

Les paramètres ventilatoires sont généralement affichés sur un écran d'affichage d'une interface graphique utilisateur (IGU), aussi appelée interface homme-machine (IHM), du ventilateur médical sous forme de courbes, de données, d'informations, d'icônes ou autres... L'IHM comprend en outre des moyens de sélection ou de réglages, telles des touches tactiles, permettant au personnel soignant d'opérer des sélections, des réglages ou autres.

Le fonctionnement du ventilateur, en particulier celui de la micro-soufflante motorisée, les affichages sur l'écran d'affichage de l'IHM... sont contrôlés et pilotés par des moyens de pilotage, typiquement une (ou des) carte électronique à microprocesseur(s) agencée dans la carcasse du ventilateur.

Beaucoup de ventilateurs comprennent un écran d'affichage intégré à la carcasse du ventilateur médical. Si ceci est un avantage en termes d'encombrement et de compacité, on s'est aperçu en pratique que le personnel soignant, par exemple les médecins ou analogues, ne sont pas toujours bien placés, par exemple dans les salles de réanimation ou analogues, pour pouvoir visualiser correctement les informations affichées par un écran d'affichage intégré.

On connait US-A-2011/0132368 qui enseigne un appareil d'assistance respiratoire comprenant des moyens de pilotage pilotant une turbine pour fournir du gaz respiratoire, les moyens de pilotage et la turbine motorisée étant agencés directement à l'intérieur d'une carcasse externe surmontée d'un écran d'affichage agencé sur un bras porteur. Dans ce cas, le bras porteur portant l'écran est directement fixé à la carcasse, ce qui peut poser des problèmes de solidité pouvant conduire à une rupture du bras sous le poids de l'écran d'affichage qu'il porte. De plus, agencer les composants internes de l'appareil directement dans la carcasse n'est pas idéal car les chocs occasionnés sur la carcasse peuvent se répercuter directement sur les composants fragiles internes de l'appareil, notamment les moyens de pilotage et la turbine motorisée, et les détériorer.

Le problème est donc de faciliter la visualisation des informations s'affichant sur l'écran d'affichage d'un ventilateur médical par le personnel soignant, en particulier dans les salles de réanimation ou analogues, qui peuvent être exiguës et donc engendrer des contraintes de positionnement du personnel soignant par rapport au ventilateur médical, tout en améliorant la solidité de l'appareil et en diminuant la sensibilité des composants fragiles internes de l'appareil, notamment en cas de chocs sur la carcasse.

Autrement, l'invention vise à proposer un ventilateur médical amélioré, en particulier de conception robuste et qui permette au personnel soignant de mieux visualiser les informations s'affichant sur l'écran d'affichage du ventilateur.

La solution de l'invention concerne alors un ventilateur médical, c'est-à-dire un appareil d'assistance respiratoire, comprenant des moyens de pilotage pilotant une soufflante motorisée (i.e. turbine ou compresseur) pour fournir du gaz respiratoire, lesdits moyens de pilotage et ladite soufflante motorisée étant agencés à l'intérieur d'une carcasse externe et un écran d'affichage.

De plus, dans ce ventilateur médical selon l'invention, les moyens de pilotage et la soufflante motorisée sont agencés sur un châssis-support rigide, agencé à l'intérieur de la carcasse du ventilateur, le châssis-support portant tout ou partie de la carcasse externe du ventilateur, ledit châssis-support comprenant en outre un bras-porteur se projetant au-dessus du châssis-support et à l'extérieur de la carcasse du ventilateur, c'est-à-dire vers le haut et extérieurement à la carcasse. Le bras-porteur porte l'écran d'affichage et comprend un système d'orientation de l'écran d'affichage agencé entre le bras-porteur et l'écran d'affichage.

Selon le mode de réalisation considéré, le ventilateur médical de l'invention peut comprendre l'une ou plusieurs des caractéristiques suivantes :
- le système d'orientation de l'écran d'affichage est configuré pour permettre d'orienter l'écran d'affichage par rapport au bras-porteur dans plusieurs directions.
- le châssis-support est formé d'une structure rigide, en particulier une structure métallique, par exemple en acier ou en alliage d'aluminium, ou, alternativement, en polymère.
- préférentiellement, le châssis-support est formé d'une structure métallique rigide en alliage d'aluminium, en particulier en alliage d'aluminium type EN AW-5754.
- le châssis-support porte en outre tout ou partie d'une carcasse périphérique externe du ventilateur.
- le châssis-support forme une armature ou squelette interne rigide portant la carcasse périphérique de sorte de renforcer la solidité de l'ensemble tout en servant d'ancrage solide au bras-porteur.
- le bras-porteur est fixé, i.e. solidarisé, au châssis-support.
- le bras-porteur forme une seconde structure rigide, de préférence métallique.
- le bras-porteur est en métal, de préférence en alliage d'aluminium, en particulier en alliage d'aluminium type EN AW-5754
- le bras-porteur est solidaire du châssis-support, c'est-à-dire fixé au châssis-support et se projette au-dessus du châssis-support en éloignement vers le haut par rapport à celui-ci.
- le bras-porteur est fixé au châssis-support par vissage, rivetage, soudage ou autre.
- la carcasse externe forme une coque protectrice autour du ventilateur.
- le système d'orientation de l'écran d'affichage est configuré pour permettre d'orienter l'écran d'affichage par rapport au bras-porteur dans plusieurs directions, i.e. des directions différentes.
- le bras-porteur forme une potence ou analogue supportant l'écran d'affichage.
- le système d'orientation est configuré pour permettre une orientation de l'écran d'affichage selon au moins deux axes (X, Y), de préférences deux axes (X, Y) perpendiculaires comprenant un axe horizontal (XX) et un axe vertical (YY).
- le système d'orientation est fixée à une extrémité libre du bras-porteur.
- le châssis-support comprend un ou plusieurs éléments de structure formant un ou des planchers, montants ou analogues préférentiellement solidarisés les uns aux autres.
- le châssis-support comprend (au moins) un élément de plancher formant un fond et des éléments de montants surmontant ledit élément de plancher.
- l'élément de plancher et les éléments de montants forment une structure de châssis tridimensionnelle portant la soufflante motorisée, les moyens de pilotage et d'autres composants internes du ventilateur médical, notamment la batterie....
- l'écran d'affichage est fixé à une platine de fixation.
- la platine de fixation est fixée au système d'orientation porté par le bras-porteur.
- la platine de fixation comprend une plaque percée d'orifices permettant le passage de vis de fixation ou analogues.
- l'écran d'affichage est fixé par vissage à la platine de fixation.
- la carcasse externe est formée plusieurs parties, i.e. plusieurs éléments de carcasse, notamment une embase et un capot.
- la carcasse externe comprend une embase formant une partie inférieure de la carcasse et dans laquelle vient se loger et se solidariser le châssis-support.
- la carcasse externe comprend un capot amovible formant une partie supérieure détachable de la carcasse.
- l'embase est fixée de manière détachable au capot.
- la carcasse comprend en outre un petit capot arrière agencé en regard de la batterie interne et démontable pour donner accès à la batterie. Le petit capot arrière se fixe par vissage sur le châssis.
- une ou des poignées de manipulation permettent de manipuler et orienter facilement l'écran d'affichage.
- il comprend deux poignées agencées de part et d'autre de l'écran, c'est-à-dire le long des côtés droit et gauche de l'écran d'affichage.
- l'écran d'affichage est protégé par un carter protecteur, c'est-à-dire qu'une enveloppe protectrice rigide est agencée autour de l'écran d'affichage.
- le carter protecteur est agencé autour de l'écran, c'est-à-dire le long des bords périphériques de l'écran et en regard de sa partie ou face arrière.
- le carter protecteur de l'écran délimite ou comprend un volume ou compartiment interne contenant les éléments électroniques servant à faire fonctionner l'écran d'affichage, tels que carte électronique, codeur rotatif, indicateur LED de mise sous tension, connexions ou autres.
- l'écran d'affichage et le carter protecteur forment une structure tridimensionnelle plate.
- les deux poignées latérales sont fixées à, i.e. ou solidaires du carter protecteur de l'écran d'affichage.
- le carter protecteur de l'écran est en polymère rigide.
- les deux poignées latérales sont en forme d'arceaux, de préférence des arceaux ayant une forme de bande.
- les deux poignées latérales et/ou le carter protecteur sont en polymère rigide, par exemple en ABS/PC.
- le système d'orientation est configuré pour permettre d'orienter l'ensemble formé par l'écran d'affichage équipé de son carter protecteur.
- le bras-porteur est protégé par une coque de protection périphérique.
- la coque de protection périphérique agencée autour du bras-porteur est traversée par au moins un câble de liaison et/ou au moins un câble électrique agencé(s) le long d'au moins une partie du bras-porteur, c'est-à-dire le ou les câbles de liaison et/ou électrique sont protégés par, i.e. agencés à l'intérieur de, la coque de protection périphérique montée autour du bras-porteur.
- la coque de protection périphérique est formée de plusieurs sous-unités, par exemple deux demi-coques assemblées l'une à l'autre de manière à prendre le bras-porteur en « sandwich ».
- la coque de protection périphérique, en particulier les sous-unités, telles les demi-coques, sont démontables indépendamment du reste, notamment de la carcasse.
- un (ou des) câble de liaison relie électriquement l'écran d'affichage aux moyens de pilotage du ventilateur, en particulier les éléments électroniques servant à faire fonctionner l'écran d'affichage, notamment une carte électronique de commande de l'affichage sur l'écran....
- un (ou des) câble électrique relie électriquement l'écran d'affichage aux moyens d'alimentation électrique.
- la coque de protection périphérique forme manchon autour du bras-porteur.
- la coque de protection périphérique s'étend entre la carcasse du ventilateur et les moyens d'orientation.
- la coque de protection périphérique agencée autour du bras-porteur et la carcasse externe agencée autour du châssis-support sont en polymère, par exemple en ABS/PC.
- les moyens de pilotage comprennent au moins une carte électronique, de préférence au moins une carte électronique montée pivotante.
- ladite au moins une carte électronique est agencée en position verticale ou quasi-verticale dans le ventilateur, lorsqu'elle est en position non-pivotée et fixée (i.e. solidarisée) au châssis-support.
- la carcasse forme une coque périphérique externe rigide protégeant les éléments et composants internes du ventilateur.
- le châssis-support porte tout ou partie de la carcasse externe du ventilateur.
- le châssis-support est fixée à l'embase, i.e. ils sont solidarisés l'un à l'autre.
- la carte électronique comprend un ou plusieurs microprocesseurs.
- la carte électronique est maintenue immobile par des moyens de maintien, de préférence des moyens de maintien par vissage.
- les moyens de maintien par vissage comprennent une ou plusieurs vis ou analogue destinées à venir se visser dans un ou des logements taraudés agencés dans la châssis-support, par exemple un ou des bras de maintien faisant partie du châssis-support, notamment deux bras de maintien agencés en parallèle.
- la carte électronique comprend une ou plusieurs expansions ou pattes de fixation comprenant chacune un orifice la traversant.
- la soufflante motorisée, i.e. turbine ou compresseur, fournit un gaz respiratoire, tel de l'air, de l'oxygène ou de l'air enrichi en oxygène.
- il comprend en outre un circuit de gaz relié fluidiquement à la soufflante de manière à acheminer le gaz fourni par ladite soufflante.
- le circuit de gaz comprend un ou des conduits, passages ou analogues pour le gaz, et de préférence une ou des vannes, notamment des électrovannes.
- la soufflante motorisée comprend un moteur électrique.
- l'écran d'affichage permet d'afficher des informations, des courbes, des touches de sélection ou de réglage, des icônes ou autres.
- il comprend en outre une interface homme-machine (IHM), de préférence l'IHM comprend l'écran d'affichage.
- l'IHM comprend en outre un ou plusieurs boutons ou touches de réglage ou sélection actionnable par l'utilisateur.
- l'écran d'affichage est tactile, de préférence il comprend une ou des touches tactiles, i.e. des touches virtuelles, s'affichant sur l'écran d'affichage.
- l'écran d'affichage (avec son carter de protection) surmonte la carcasse externe du ventilateur lorsqu'il est dans sa position normale d'utilisation, c'est-à-dire avec embase posée et/ou en regard d'une surface support, tel un chariot hospitalier, un meuble, le sol ou autre.
- l'écran (avec son carter de protection) est configuré pour être détachable et pour pouvoir être suspendu sur un autre support externe, c'est-à-dire ailleurs que sur le ventilateur médical, par exemple il comprend une interface ou platine de type VESA.
- l'interface ou platine de type VESA est monté sur la carter protecteur, en particulier sur sa face arrière située derrière l'écran.
- les moyens de pilotage, en particulier la carte électronique à microprocesseur(s), sont configurés pour piloter la soufflante, en particulier le moteur électrique, et/ou pour commander l'affichage sur l'écran.
- il comprend en outre des moyens d'alimentation en courant électrique.
- les moyens d'alimentation en courant électrique sont raccordés électriquement (directement ou indirectement) à la carte électronique, à l'écran d'affichage, à la soufflante et plus généralement à tous les éléments ou composants du ventilateur requérant du courant électrique pour fonctionner.
- les moyens d'alimentation en courant électrique comprennent une batterie rechargeable.
- les moyens d'alimentation en courant électrique comprennent des moyens de raccordement au secteur (110/220 V), notamment un ou des câbles électriques et/ou une ou des prises de raccordement.
- le châssis-support porte en outre un ou plusieurs ventilateurs de refroidissement servant à faire circuler de l'air dans la carcasse afin de refroidir la ou les cartes électroniques, voire d'autres composants internes du ventilateur, comme le moteur de la turbine ou encore des blocs échangeurs thermiques favorisant les échanges thermiques de manière à ce que le gaz envoyé au patient ne soit pas trop chaud, par exemple qu'il n'excède pas 70°C sur une plage de 120 secondes ou une énergie équivalente dans des conditions d'environ 43°C à 100% d'humidité, soit une enthalpie spécifique de 197 kj/m³ quelle que soit la plage de ventilation choisie.
- le châssis-support porte en outre au moins une partie du circuit de gaz (i.e. conduits, vannes...).
- le châssis porte en outre une carte d'alimentation électronique servant à convertir la tension du secteur (110/220V) en une tension d'alimentation adéquate compatible avec les composants électroniques et autres du ventilateur.
- le châssis-support porte en outre la batterie d'alimentation électrique rechargeable.
- les moyens de pilotage sont configurés pour commander la soufflante de manière à délivrer du gaz au moins pendant les phases inspiratoires du patient.
- les moyens de pilotage sont configurés pour piloter les accélérations et les freinages/ralentissements du moteur électrique de la soufflante.
- l'écran d'affichage comprend une ou des touches tactiles permettant d'opérer des sélections, des validations, des réglages, des démarrages ou des arrêts de fonctionnement... Les touches tactiles sont à actionnement digital, c'est-à-dire qu'elles sont activées lorsque l'utilisateur appuie dessus avec son doigt, typiquement son index.
- l'écran d'affichage est configuré pour opérer un affichage d'informations sous forme de caractères alphanumériques, de représentations graphiques (e.g. graphes, courbes, dessins, icones, etc....), de photos, d'animations vidéo...... ou autres.
- la soufflante comprend un moteur électrique, c'est-à-dire fonctionnant grâce à un courant électrique.
- la soufflante comprend un moteur électrique entraînant une roue à ailettes agencée dans le compartiment interne d'une volute.
- la soufflante est commandée pour atteindre une vitesse de rotation maximale de 25 000 tours/minute, typiquement entre 500 et 15 000 tr/min, par exemple de l'ordre de 9 500 tr/min maximum.

L'invention va maintenant être mieux comprise grâce à la description détaillée suivante, faite à titre illustratif mais non limitatif, en référence aux figures annexées parmi lesquelles :
Fig. 1 est un mode de réalisation d'un ventilateur médical selon l'invention.
Fig. 2 est une vue par transparence du ventilateur médical de Fig. 1.
Fig. 3 représente le ventilateur médical de Fig. 1 sans son capot supérieur.
Fig. 4 est une vue sous un autre angle du ventilateur médical de Fig. 3.
Fig. 5 représente le ventilateur médical de Fig. 1 sans son capot supérieur et sans l'écran d'affichage.
Fig. 6 est une vue sous un autre angle du ventilateur médical de Fig. 5.
Fig. 7 est une vue latérale du ventilateur médical de Fig. 5.

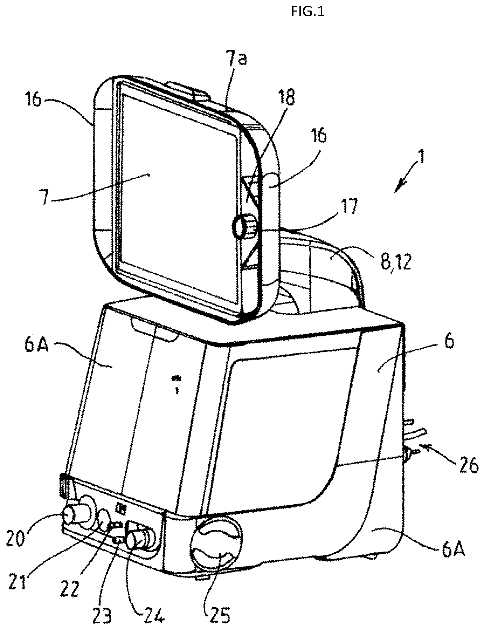

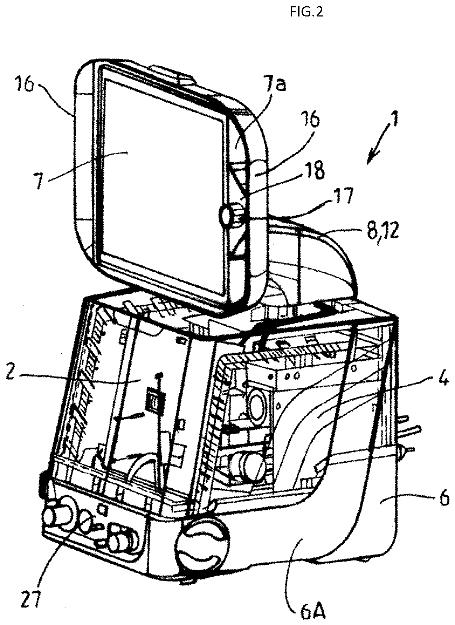

Fig. 1 à Fig. 7 schématise un mode de réalisation d'un ventilateur médical 1, c'est-à-dire un appareil d'assistance respiratoire, délivrant une ventilation assistée à un patient selon l'invention. Il est conçu pour fournir un gaz respiratoire à un patient, à savoir un gaz médical, par exemple de l'air provenant de l'atmosphère ambiante, de l'oxygène pur provenant d'une source d'oxygène, par exemple une bouteille d'oxygène médical ou d'une canalisation véhiculant de l'oxygène médical au sein d'un bâtiment hospitalier, ou encore de l'air enrichi en oxygène.

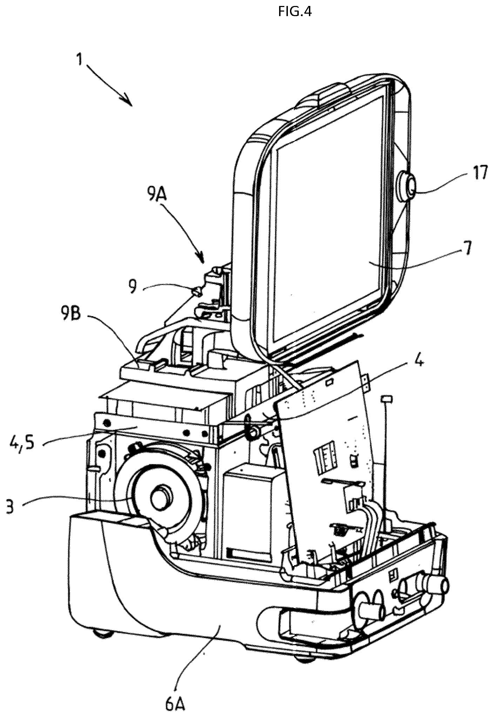

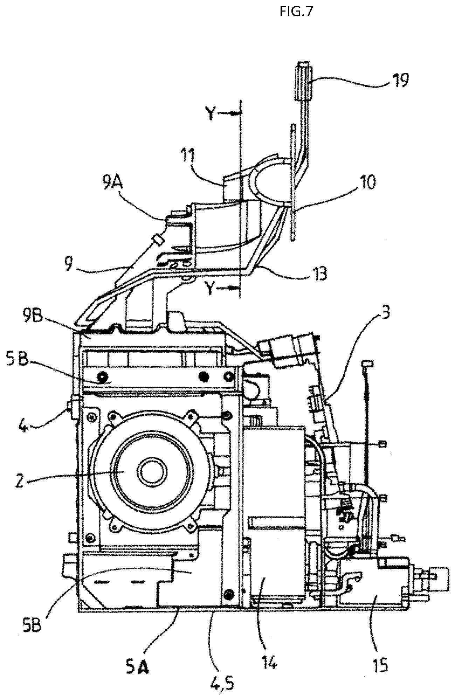

Le ventilateur médical 1 comprend une carcasse ou coque externe 6 rigide, par exemple en polymère, dans laquelle sont agencés les différents éléments permettant son fonctionnement, en particulier une soufflante 2 motorisée (cf. Fig. 4 et Fig. 7), d'environ 20 cm de diamètre, aussi appelée turbine ou compresseur, délivrant le gaz respiratoire (e.g. air, un mélange air/O₂, oxygène pur...), un circuit de gaz interne (i.e. conduits ou passages de gaz, vannes...) pour véhiculer les flux gazeux dans le ventilateur 1, des moyens de pilotage 2 à microprocesseur(s), une (ou des) batterie ou analogue rechargeable...

Lorsqu'un mélange air/O₂ est désiré, il peut être réalisé dans un mélangeur de gaz interne du ventilateur 1, aussi appelé bloc mélangeur 14, comprenant des entrées ou admissions d'air et d'O₂ (à la pression désirée) et une sortie ou refoulement du mélange gazeux air/O₂ ainsi obtenu.

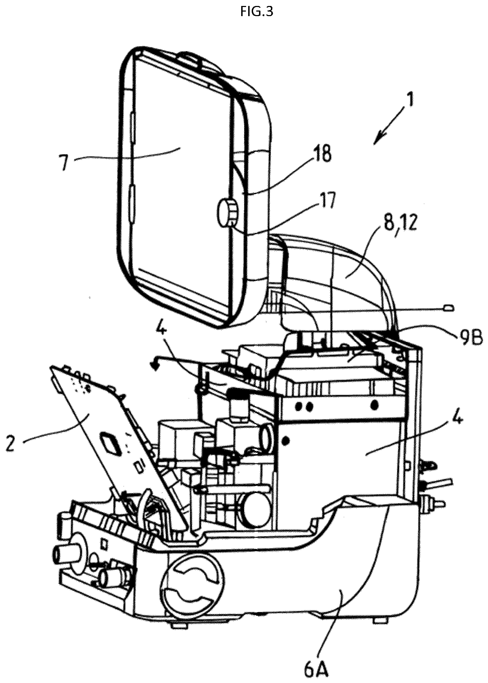

Dans ce mode de réalisation, la carcasse 6 est formée de deux sous-unités (ou plus) venant se solidariser l'une à l'autre, à savoir une embase 6A formant la partie basse ou inférieure de la carcasse 6 et un capot 6B formant la partie haute ou supérieure de la carcasse 6. Comme illustré en Fig. 3 à Fig. 7, le capot 6B est amovible, c'est-à-dire peut être retiré pour donner accès à l'intérieur du ventilateur 1, notamment lors de son assemblage (i.e. fabrication) ou lors de sa maintenance (i.e. entretien).

En fonctionnement, l'air ambiant est aspiré par la soufflante 3 motorisée, i.e. la turbine ou compresseur, via un port d'entrée en communication fluidique avec l'atmosphère ambiante. De préférence, l'air est filtré avant de pénétrer dans la soufflante.

La soufflante motorisée 3 a une architecture classique. Elle comprend une (ou des) roue à ailettes entrainée en rotation par l'axe ou arbre rotatif d'un moteur électrique. La roue à ailettes est agencée, mobile en rotation, au sein d'un compartiment à roue agencé dans une volute, de préférence prise en sandwich entre une demi-volte inférieure et une demi-volte supérieure fixée de manière étanche l'une à l'autre. La volute comprend une entrée pour l'air provenant du port d'entrée d'air, c'est-à-dire l'air aspiré par la roue à ailettes, lors de ses rotations. Par ailleurs, la volute de la soufflante comprend un conduit de sortie de gaz par lequel le débit de gaz est expulsé hors de la volute, pendant les rotations de la roue à ailettes, et acheminé jusqu'au patient, via un conduit de gaz flexible débouchant au niveau d'une interface respiratoire patient, tel un masque respiratoire ou une sonde trachéale. Le conduit flexible vient se raccorder fluidiquement de manière détachable, au ventilateur 1.

Selon un mode de réalisation, le fonctionnement de la soufflante 2, en particulier les accélérations ou décélérations/freinages du moteur, est commandé par des moyens de pilotage 3, aussi appelés unité de commande électronique, typiquement une (ou des) carte électronique à microprocesseur(s), de manière à fournir le gaz respiratoire aux débit et pression désirés, de préférence sélectionnés ou réglés par l'utilisateur via l'IHM, comme expliqué ci-après.

Selon un autre mode de réalisation, la vitesse du moteur n'est pas commandée pour atteindre le débit gazeux nécessaire. Ce sont des électrovannes qui sont impliqués dans la régulation et qui définissent les volumes et les pressions désirés. Dans ce cas, seules deux vitesses de turbine sont mises en œuvre.

Par ailleurs, le ventilateur 1 peut comprendre en outre d'autres éléments, en particulier un (ou des) capteur de pression ou un (ou des) capteur de débit (e.g. capteur de débit massique et/ou à fil chaud) pour mesurer la pression du gaz et/ou le débit du flux gazeux dans le circuit de gaz interne, typiquement en aval de la soufflante. Les mesures (i.e. signaux) de pression et de débit sont transmises par les capteurs aux moyens de pilotage 2 du ventilateur 1.

Les moyens de pilotage 2 comprennent un ou plusieurs microprocesseurs agencés sur la (ou des) carte électronique, avantageusement un microcontrôleur, mettant en œuvre un ou des algorithmes, permettant de monitorer et réguler la ventilation délivrée au patient en exploitant notamment les signaux de pression et de débit mesurés par les capteurs de pression et/ou de débit.

La (ou les) carte électronique peut aussi comprendre des moyens de mémorisation pour mémoriser notamment les valeurs de pression et/ou de débit, par exemple une mémoire type flash ou autre portée par la carte électronique, ou dans tout autre moyen de mémorisation, y compris dans un algorithme du microprocesseur.

Par ailleurs, le ventilateur 1 comprend aussi une interface homme-machine ou IHM servant d'interface de réglage ou de sélection pour l'utilisateur, i.e. le personnel soignant.

Comme illustré sur les Fig. 1 à Fig. 4, l'IHM comprend ici un écran d'affichage 7 surmontant la carcasse 6 du ventilateur 1.

Selon l'invention, l'écran d'affichage 7 est porté par un bras-porteur 9 formant un mât ou une potence supportant l'écran d'affichage 7.

L'écran d'affichage 7 est protégé car un carter protecteur 7a en polymère rigide, agencé autour de l'écran, c'est-à-dire le long des bords périphériques de l'écran 7 et en regard de sa partie ou face arrière. Il délimite un compartiment interne contenant les éléments électroniques servant à faire fonctionner l'écran d'affichage, tels que carte électronique, codeur rotatif, indicateur LED de mise sous tension ou autre.

L'écran d'affichage 7 a préférentiellement une forme rectangulaire. Le carter protecteur 7a forme ou comprend le long des bords périphériques de l'écran 7, c'est-à-dire à la périphérie de l'écran 7, un cadre 18 rigide entourant l'écran 7. L'écran d'affichage 7 et le carter protecteur 7a forment une structure tridimensionnelle plate, couramment appelé « écran plat ».

Afin de permettre une orientation facile et selon plusieurs axes de l'écran d'affichage 7 (et bien entendu de son carter 7a), un système d'orientation 11 de l'écran d'affichage 7 est agencé entre le bras-porteur 9 et l'écran d'affichage 7 de manière à pouvoir orienter et/ou positionner l'écran d'affichage 7 dans l'espace et permettre à un personnel soignant de pouvoir bien distinguer les informations qui s'y affichent. Autrement dit, le système d'orientation 11 est agencé à l'extrémité libre 9A du bras-porteur 9 et pris en sandwich entre ledit bras-porteur 9 et l'écran d'affichage 7.

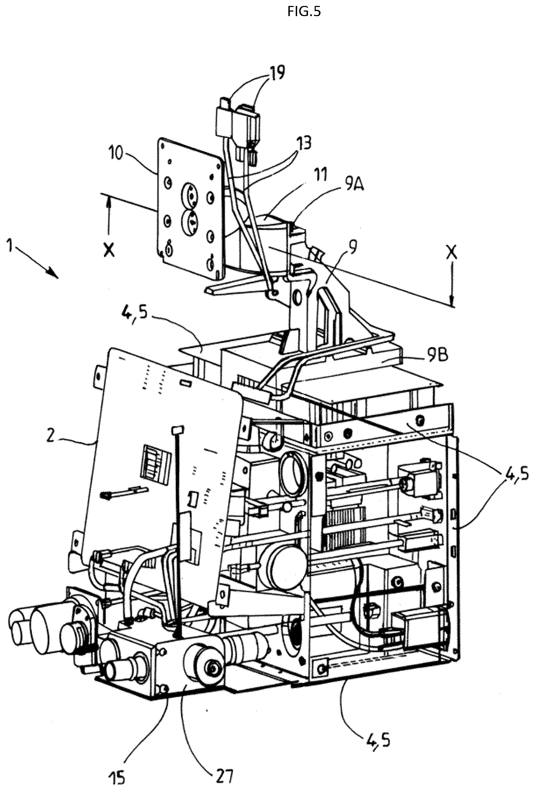

Préférentiellement, le système d'orientation 11 de l'écran 7 est configuré pour être orientable selon plusieurs axes par rapport au bras-porteur 9, typiquement au moins 2 axes perpendiculaires (X, Y), à savoir ici un axe horizontal (XX) et un axe vertical (YY), tel que représenté en Fig. 5 et Fig. 7.

Ici, le système d'orientation de l'écran 11 comprend une platine 10 à 2 axes (par exemple de type VESA) ou analogue, tel qu'illustré en Fig. 5 à Fig. 7. La platine 10 comprend ici une plaque percé d'orifices de passage de vis afin de pouvoir y fixer l'écran 7 par vissage, en particulier de pouvoir la fixer par vissage à la face arrière du carter 7a qui protège l'écran numérique 7. Toutefois, un autre système pourrait être utilisé comme par exemple un système à rotule.

Le bras-porteur 9 est fixé, par une extrémité 9B, à un châssis-support 4 interne au ventilateur 1, c'est-à-dire agencé à l'intérieur de la carcasse périphérique externe 6 du ventilateur 1. Comme on le voit, le bras-porteur 9 se projette au-dessus du châssis-support 4, c'est-à-dire fait saillie à l'extérieur et au-dessus de la carcasse 6 du ventilateur 1 de sorte que l'écran 7 soit situé au-dessus de ladite carcasse 6.

L'orientation et les manipulations de l'écran 7 se font via deux poignées latérales 16, par exemple en forme d'arceaux ou autres, agencées de part et d'autre de l'écran 7, c'est-à-dire le long des bords droit et gauche de l'écran 7, comme illustré en Fig. 1. Les deux poignées latérales 16 sont agencées ici sensiblement parallèles l'une à l'autre.

Les deux poignées latérales 16 sont par exemple fixées au carter protecteur 7a de l'écran d'affichage 7. Dans le mode de réalisation présenté, elles sont en forme d'arceaux, de préférence des arceaux ayant une forme de bande. Bien entendu, elles pourraient avoir une autre forme.

Préférentiellement, les deux poignées latérales 16 et le carter protecteur 7a sont en polymère rigide, par exemple en ABS/PC.

Par ailleurs, le châssis-support 4 sert aussi de support à d'autres éléments ou composants du ventilateur 1, comme les moyens de pilotage 2, typiquement la carte électronique, la soufflante motorisée 3, la batterie....

Le châssis-support 4 est formé d'une structure métallique rigide 5, préférentiellement métallique, notamment en tôle d'acier ou en alliage d'aluminium, de préférence en alliage d'aluminium. Par exemple, on peut utiliser un alliage d'aluminium de type EN AW-5754. En effet, utiliser un tel alliage d'aluminium est avantageux car léger, solide, peu cher, sujet à une corrosion limitée, conducteur électrique permettant de faire une liaison équipotentielle entre les composants et la terre...

Cette structure métallique rigide 5 formant le châssis-support 4 comprend un ou plusieurs éléments de structure formant un ou des planchers, montants ou analogues solidarisés les uns aux autres en une structure tridimensionnelle portant la soufflante motorisée 3, les moyens de pilotage 2 et d'autres composants internes du ventilateur médical 1, notamment la batterie...

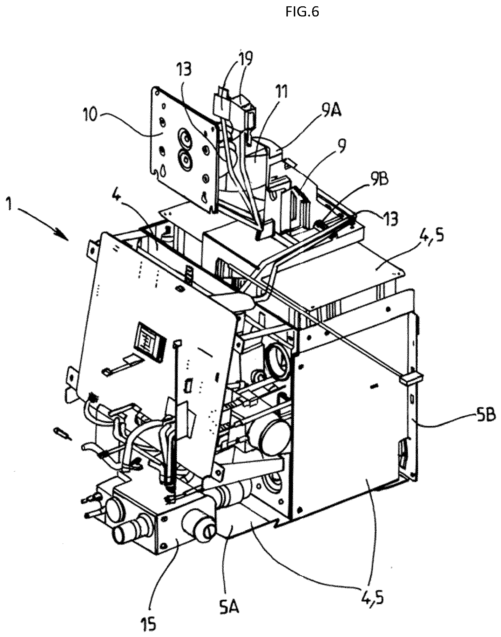

Préférentiellement, la structure métallique rigide 5 du châssis-support 4 comprend un (ou des) élément de plancher formant un fond 5A et des éléments de montants 5B surmontant ledit élément de plancher 5A, comme illustré en Fig. 6 et Fig. 7, de sorte de former ladite structure tridimensionnelle formant une armature ou un squelette interne au ventilateur 1. Les éléments de plancher et de montants 5A, 5B peuvent être des plaques, des parois ou autres.

Le châssis-support 4 permet aussi de fixer et porter la carcasse externe 6 du ventilateur 1, laquelle comprend ici une embase 6A et un capot amovible 6B. L'embase 6A forme la partie inférieure de la carcasse 6 dans laquelle vient de loger et se solidariser le châssis-support 4, alors que le capot amovible 6B forme une partie supérieure détachable de la carcasse 6, laquelle peut se détacher du ventilateur 1 pour donner accès à l'intérieur du ventilateur 1, notamment lors du montage/fabrication ou ultérieurement lors d'opérations de maintenance, réparation ou analogue, comme illustré en Fig. 3 qui montre le ventilateur 1 sans le capot 6B.

Par ailleurs, le bras-porteur 9 qui est fixé au châssis-support 4, par exemple par vissage, est protégé par une coque de protection périphérique 12 formant un manchon autour dudit bras-porteur 9.

Cette coque de protection périphérique 12 s'étend sensiblement entre la carcasse externe 6 du ventilateur 1, en particulier le haut du capot amovible 6B, et le système d'orientation 11, comme visible sur Fig. 1 à Fig. 3.

La coque de protection périphérique est avantageusement formée de plusieurs sous-unités ou parties fixées les unes aux autres, par exemple deux demi-coques venant se coupler l'une à l'autre en prenant en sandwich la structure métallique formant le bras-porteur 9. Elles sont démontables indépendamment du reste, notamment de la carcasse.

La coque de protection périphérique 12 est en polymère ou tout autre matériau adapté.

La coque de protection périphérique 12 est en outre traversée par des câbles de liaison 13 qui sont agencés le long du bras-porteur 9, comme illustré sur Fig. 5 à Fig. 7. Ces câbles de liaison 13 électrique relient notamment les moyens de pilotage 2 et les moyens d'alimentation électrique à l'écran 7 afin de l'alimenter en courant et de piloter les affichages qui s'opèrent sur ledit écran 7.

Préférentiellement, les câbles de liaison 13 sont fixés au bras-porteur 9 afin d'être maintenus dans la coque de protection périphérique 12. Ces câbles de liaison 13 sont munis de connecteurs 19 de raccordement électrique et mécanique pour permettre leur connexion à l'écran 7.

L'écran d'affichage 7 est préférentiellement un écran tactile, et de préférence en couleurs, sur lequel s'affichent, pendant son fonctionnement, sur des touches virtuelles de sélection ou de réglage actionnables par action digitale d'un utilisateur, c'est-à-dire lorsque l'utilisateur appuie dessus avec son index par exemple.

Les touches virtuelles peuvent se présenter sous forme d'icônes ou analogues. Elles permettent à un utilisateur d'opérer des choix et/ou des réglages, par exemple des niveaux de pression souhaités ou d'autres paramètres ventilatoires, d'activer et de désactiver des options de ventilation 1, ou des alarmes.

On voit que l'IHM comprend aussi ici un (ou des) bouton 17 rotatif qui sert à faire varier les valeurs à régler et à valider volontairement les configurations choisies. Il est agencé à côté de l'écran 7, c'est-à-dire dire sur le cadre 18 rigide du carter 7a entourant l'écran 7. Bien entendu, on peut prévoir aussi d'autres moyens de réglage/sélection comme par exemple des touches, curseurs ou analogues.

L'écran numérique tactile 7, en particulier les éléments électroniques servant à faire fonctionner l'écran d'affichage contenus dans le compartiment interne délimité par le carter 7a, est (sont) relié(s) électriquement aux moyens de pilotage 2 de sorte que ces derniers puissent commander/piloter les affichages se faisant sur l'écran 7.

Une source de courant électrique, par exemple une batterie rechargeable intégrée au ventilateur médical 1, un ou des câbles 26 avec prise de raccordement au secteur (110/220V), ou les deux, et préférentiellement une carte d'alimentation faisant office de convertisseur de courant/tension, permet d'alimenter en énergie électrique, les composants du ventilateur 1 en ayant besoin pour fonctionner, en particulier la soufflante, les moyens de pilotage 2, notamment la carte(s) électronique(s) et le(s) microprocesseur(s) porté par la carte, les capteurs de pression et/ou de débit, l'écran d'affichage 7, et/ou d'autres composants.

Afin de faciliter les opérations de montage et d'entretien et/ou maintenance de la carte électronique au sein du ventilateur 1, la carte électronique des moyens de pilotage 2 est montée pivotante sur le châssis-support 4, comme illustré sur Fig. 3. L'angle de pivotement *β* de la carte électronique est inférieur à 150°, en général inférieur à 120°, typiquement compris entre 20° et 90°, par exemple de l'ordre de 30° à 60°.

Le ventilateur médical 1 comprend par ailleurs :
- une connexion de valve expiratoire 20 pour y raccorder fluidiquement une valve expiratoire, telle la Valve Monnal'EVA^{™}, servant à mesurer le débit de gaze expiré par le patient et rejeté à l'atmosphère,
- un bouton d'éjection de valve expiratoire 21 servant à désolidariser la valve du ventilateur 1, par exemple lors de sa maintenance ou autre,
- un connecteur de raccordement d'un dispositif de nébulisation 22 pour y raccorder fluidiquement un dispositif de nébulisation servant à apporter un médicament à nébuliser qui est mélangé au flux gazeux respiratoire au sein du ventilateur 1,
- un connecteur de raccordement de sonde 23 pour y raccorder fluidiquement une sonde œsophagienne permettant de mesure la pression interne à partir d'une telle sonde,
- un connecteur de circuit patient 24 pour y raccorder le circuit patient servant à acheminer les flux gazeux, notamment vers le patient.
- un logement à sonde FiO₂ 25 et son couvercle d'obturation pour y ranger une sonde de mesure du taux de O₂ sanguin du patient.

Par ailleurs, le ventilateur 1 peut comprendre aussi d'autres éléments, notamment des connecteurs servant à différents raccordements de type prise USB double, connecteur ADMI, connecteurs RS235 SUB D5 et RJ45, ou autres, un haut-parleur, une sonde de température, un bloc inspiratoire 27 porté par le châssis 4 comprenant les raccordements du circuit patient, de la sortie expiratoire, de la sonde FiO₂, de la sonde de température, du raccord de nébulisation, du raccord de la sonde oesophagiale, des piquages de mesure de pression... un bloc central comprenant les électrovannes, l'admission d'O₂, le détendeur de nébulisation et le capteur de pression O₂...., des tubulures reliant les piquages de mesure de pression vers les capteurs de pression embarqués sur la carte électronique, ou d'autres éléments.

D'une façon générale, le ventilateur médical de l'invention est particulièrement bien adapté à une utilisation en milieu hospitalier.

## Revendications

1. Ventilateur médical (1) comprenant :
- des moyens de pilotage (2) pilotant une soufflante motorisée (3) pour fournir du gaz respiratoire, lesdits moyens de pilotage (2) et ladite soufflante motorisée (3) étant agencés à l'intérieur d'une carcasse externe (6), et
- un bras-porteur (9) se projetant à l'extérieur de la carcasse (6) du ventilateur (1), le bras-porteur (9) portant un écran d'affichage (7) et comprenant un système d'orientation (11) de l'écran d'affichage (7) agencé entre le bras-porteur (9) et l'écran d'affichage (7),
**caractérisée en ce que** :
- les moyens de pilotage (2) et la soufflante motorisée (3) sont agencés sur un châssis-support (4) rigide agencé à l'intérieur de la carcasse (6) du ventilateur (1),
- le châssis-support (4) porte en outre tout ou partie de la carcasse externe (6) du ventilateur (1), et
- le châssis-support (4) comprend le bras-porteur (9) se projetant au-dessus du châssis-support (4).

2. Ventilateur selon la revendication 1, **caractérisé en ce que** le châssis-support (4) forme une structure métallique rigide (5).

3. Ventilateur selon l'une des revendications précédentes, **caractérisé en ce que** le châssis-support (4) comprend au moins un élément de plancher formant un fond et des éléments de montants surmontant ledit élément de plancher.

4. Ventilateur selon la revendication 3, **caractérisé en ce que** l'élément de plancher et les éléments de montants forment une structure de châssis tridimensionnelle portant la soufflante motorisée (3) et les moyens de pilotage (2) du ventilateur médical (1).

5. Ventilateur selon la revendication 1, **caractérisé en ce que** le système d'orientation (11) de l'écran d'affichage (7) est configuré pour permettre d'orienter l'écran d'affichage (7) par rapport au bras-porteur (9) dans plusieurs directions.

6. Ventilateur selon l'une des revendications 1 ou 5, **caractérisé en ce que** le système d'orientation (11) est configuré pour permettre une orientation de l'écran d'affichage (7) selon au moins deux axes (X, Y), de préférences deux axes (X, Y) perpendiculaires comprenant un axe horizontal (XX) et un axe vertical (YY).

7. Ventilateur selon l'une des revendications 1, 5 ou 6, **caractérisé en ce que** le système d'orientation (11) est fixée à une extrémité libre (9A) du bras-porteur (9).

8. Ventilateur selon l'une des revendications 1 ou 7, **caractérisé en ce que** l'écran d'affichage (7) est fixé à une platine de fixation (10), ladite platine de fixation (10) étant fixée au système d'orientation (11) porté par le bras-porteur (9).

9. Ventilateur selon la revendication 1, **caractérisé en ce que** la carcasse externe (6) comprend :
- une embase (6A) formant une partie inférieure de la carcasse (6) et dans laquelle vient de loger et se solidariser le châssis-support (4), et/ou
- un capot amovible (6B) formant une partie supérieure détachable de la carcasse (6).

10. Ventilateur selon la revendication 1, **caractérisé en ce que** le bras-porteur (9) est protégé par une coque de protection périphérique (12).

11. Ventilateur selon la revendication 10, **caractérisé en ce que** la coque de protection périphérique (12) agencée autour du bras-porteur (9) est traversée par au moins un câble de liaison (13) agencé le long d'au moins une partie du bras-porteur (9).

12. Ventilateur selon l'une des revendications 1 ou 2, **caractérisé en ce que** le bras-porteur (9) et le châssis-support (4) sont en alliage d'aluminium.

13. Ventilateur selon la revendication 1, **caractérisé en ce que** l'écran (7) comprend une ou des poignées de manipulation (16) permettent de manipuler et orienter l'écran d'affichage (7), de préférence la ou les poignées latérales (16) sont en forme d'arceaux

14. Ventilateur selon l'une des revendications 1 ou 13, **caractérisé en ce qu'**il comprend deux poignées (16) agencées de part et d'autre de l'écran (7).

15. Ventilateur selon l'une des revendications 13 ou 14, **caractérisé en ce que** la ou les poignées latérales (16) sont fixées à un carter protecteur (7a) protégeant l'écran (7).

## Patentansprüche

1. Ein medizinisches Beatmungsgerät (1), umfassend:
- Steuerungsmittel (2), die ein motorisiertes Gebläse (3) steuern, um Atemgas zu liefern, wobei die besagten Steuerungsmittel (2) und das besagte motorisierte Gebläse (3) im Inneren eines externen Gehäuses (6) angeordnet sind, und
- einen Tragarm (9), der aus dem Gehäuse (6) des Beatmungsgeräts (1) herausragt, wobei der Tragarm (9) einen Anzeigebildschirm (7) trägt und ein Ausrichtungssystem (11) des Anzeigebildschirms (7) umfasst, das zwischen dem Tragarm (9) und dem Anzeigebildschirm (7) angeordnet ist,
**dadurch gekennzeichnet, dass**:
- die Steuerungsmittel (2) und das motorisierte Gebläse (3) auf einem starren Trägerchassis (4) angeordnet sind, das im Inneren des Gehäuses (6) des Beatmungsgeräts (1) angeordnet ist,
- das Trägerchassis (4) ferner das gesamte oder einen Teil des externen Gehäuses (6) des Beatmungsgeräts (1) trägt, und
- das Trägerchassis (4) den Tragarm (9) umfasst, der sich über das Trägerchassis (4) erstreckt.

2. Beatmungsgerät nach Anspruch 1, **dadurch gekennzeichnet, dass** das Trägerchassis (4) eine starre metallische Struktur (5) bildet.

3. Beatmungsgerät nach einem der vorhergehenden Ansprüche, **dadurch gekennzeichnet, dass** das Trägerchassis (4) mindestens ein ein Boden bildendes Bodenelement und auf dem besagten Bodenelement aufliegende Ständerelemente umfasst.

4. Beatmungsgerät nach Anspruch 3, **dadurch gekennzeichnet, dass** das Bodenelement und die Ständerelemente eine dreidimensionale Chassis-Struktur bilden, die das motorisierte Gebläse (3) und die Steuerungsmittel (2) des medizinischen Beatmungsgeräts (1) trägt.

5. Beatmungsgerät nach Anspruch 1, **dadurch gekennzeichnet, dass** das Ausrichtungssystem (11) des Anzeigebildschirms (7) so konfiguriert ist, dass es die Ausrichtung des Anzeigebildschirms (7) in Bezug auf den Tragarm (9) in mehreren Richtungen ermöglicht.

6. Beatmungsgerät nach einem der Ansprüche 1 oder 5, **dadurch gekennzeichnet, dass** das Ausrichtungssystem (11) so konfiguriert ist, dass es eine Ausrichtung des Anzeigebildschirms (7) nach mindestens zwei Achsen (X, Y) ermöglicht, vorzugsweise zwei senkrechten Achsen (X, Y), die eine horizontale Achse (XX) und eine vertikale Achse (YY) umfassen.

7. Beatmungsgerät nach einem der Ansprüche 1, 5 oder 6, **dadurch gekennzeichnet, dass** das Ausrichtungssystem (11) an einem freien Ende (9A) des Tragarms (9) befestigt ist.

8. Beatmungsgerät nach einem der Ansprüche 1 oder 7, **dadurch gekennzeichnet, dass** der Anzeigebildschirm (7) an einer Befestigungsplatte (10) befestigt ist, wobei die besagte Befestigungsplatte (10) an dem vom Tragarm (9) getragenen Ausrichtungssystem (11) befestigt ist.

9. Beatmungsgerät nach Anspruch 1, **dadurch gekennzeichnet, dass** das externe Gehäuse (6) umfasst:
- eine Basis (6A), die einen unteren Teil des Gehäuses (6) bildet und in der das Trägerchassis (4) untergebracht und befestigt ist, und/oder
- eine abnehmbare Haube (6B), die einen oberen abnehmbaren Teil des Gehäuses (6) bildet.

10. Beatmungsgerät nach Anspruch 1, **dadurch gekennzeichnet, dass** der Tragarm (9) durch eine periphere Schutzschale (12) geschützt ist.

11. Beatmungsgerät nach Anspruch 10, **dadurch gekennzeichnet, dass** die um den Tragarm (9) angeordnete periphere Schutzschale (12) von mindestens einem Verbindungskabel (13) durchquert wird, das entlang mindestens eines Teils des Tragarms (9) angeordnet ist.

12. Beatmungsgerät nach einem der Ansprüche 1 oder 2, **dadurch gekennzeichnet, dass** der Tragarm (9) und das Trägerchassis (4) aus einer Aluminiumlegierung bestehen.

13. Beatmungsgerät nach Anspruch 1, **dadurch gekennzeichnet, dass** der Bildschirm (7) einen oder mehrere Handhabungsgriffe (16) zum Handhaben und Ausrichten des Anzeigebildschirms (7) umfasst, wobei vorzugsweise der oder die seitlichen Griffe (16) in Form von Bügeln ausgebildet sind.

14. Beatmungsgerät nach einem der Ansprüche 1 oder 13, **dadurch gekennzeichnet, dass** es zwei Griffe (16) umfasst, die auf beiden Seiten des Bildschirms (7) angeordnet sind.

15. Beatmungsgerät nach einem der Ansprüche 13 oder 14, **dadurch gekennzeichnet, dass** der oder die seitlichen Griffe (16) an einem Schutzgehäuse (7a) befestigt sind, das den Bildschirm (7) schützt.

## Claims

1. A medical ventilator (1) comprising:
- control means (2) for controlling a motorized blower (3) to supply respiratory gas, said control means (2) and said motorized blower (3) being arranged inside an external casing (6), and
- a support arm (9) projecting to the outside of the casing (6) of the ventilator (1), the support arm (9) carrying a display screen (7) and comprising an orientation system (11) for the display screen (7) arranged between the support arm (9) and the display screen (7),
**characterized in that**:
- the control means (2) and the motorized blower (3) are arranged on a rigid support frame (4) arranged inside the casing (6) of the ventilator (1),
- the support frame (4) further carries all or part of the external casing (6) of the ventilator (1), and
- the support frame (4) comprises the support arm (9) projecting above the support frame (4).

2. The ventilator according to claim 1, **characterized in that** the support frame (4) forms a rigid metallic structure (5).

3. The ventilator according to any one of the preceding claims, **characterized in that** the support frame (4) comprises at least one floor element forming a bottom and upright elements surmounting said floor element.

4. The ventilator according to claim 3, **characterized in that** the floor element and the upright elements form a three-dimensional frame structure carrying the motorized blower (3) and the control means (2) of the medical ventilator (1).

5. The ventilator according to claim 1, **characterized in that** the orientation system (11) of the display screen (7) is configured to allow the orientation of the display screen (7) relative to the support arm (9) in several directions.

6. The ventilator according to any one of claims 1 or 5, **characterized in that** the orientation system (11) is configured to allow an orientation of the display screen (7) along at least two axes (X, Y), preferably two perpendicular axes (X, Y) comprising a horizontal axis (XX) and a vertical axis (YY).

7. The ventilator according to any one of claims 1, 5 or 6, **characterized in that** the orientation system (11) is fixed to a free end (9A) of the support arm (9).

8. The ventilator according to any one of claims 1 or 7, **characterized in that** the display screen (7) is fixed to a mounting plate (10), said mounting plate (10) being fixed to the orientation system (11) carried by the support arm (9).

9. The ventilator according to claim 1, **characterized in that** the external casing (6) comprises:
- a base (6A) forming a lower part of the casing (6) and in which the support frame (4) is housed and secured, and/or
- a removable cover (6B) forming an upper detachable part of the casing (6).

10. The ventilator according to claim 1, **characterized in that** the support arm (9) is protected by a peripheral protective shell (12).

11. The ventilator according to claim 10, **characterized in that** the peripheral protective shell (12) arranged around the support arm (9) is traversed by at least one connecting cable (13) arranged along at least a part of the support arm (9).

12. The ventilator according to any one of claims 1 or 2, **characterized in that** the support arm (9) and the support frame (4) are made of aluminum alloy.

13. The ventilator according to claim 1, **characterized in that** the screen (7) comprises one or more handling grips (16) for handling and orienting the display screen (7), preferably the one or more side grips (16) are in the form of arches

14. The ventilator according to any one of claims 1 or 13, **characterized in that** it comprises two grips (16) arranged on either side of the screen (7).

15. The ventilator according to any one of claims 13 or 14, **characterized in that** the one or more side grips (16) are fixed to a protective housing (7a) protecting the screen (7).
